# EUROPÄISCHE PATENTSCHRIFT

(11) **EP 0 923 904 B1**
(45) Veröffentlichungstag und Bekanntmachung des Hinweises auf die Patenterteilung: **26.11.2003**
(21) Anmeldenummer: 98121061.0
(22) Anmeldetag: 06.11.1998
(51) Int. Cl.: A61B 6/04

(54) **Vorrichtung zum Bewegen eines Röntgentischs oder dgl.**
Device for moving an x-ray table or the like
Dispositif pour déplacer une table pour rayons X ou similaire

(30) Priorität: 19.12.1997 DE 19756783
(43) Veröffentlichungstag der Anmeldung: 23.06.1999
(73) Patentinhaber: Hans Pausch GmbH & Co., 91056 Erlangen (DE)
(72) Erfinder: Mattern, Helmut, 91091 Grossenseebach (DE)
(74) Vertreter: Gassner, Wolfgang, Dr.

(56) Entgegenhaltungen:
- US-A- 3 783 282
- US-A- 4 912 754
- US-A- 4 960 271
- US-A- 5 042 487

## Beschreibung

Die Erfindung betrifft eine Vorrichtung zum Bewegen eines Röntgentischs oder dgl.

Aus dem im Oktober 1996 erschienenen Prospekt "UROMAT 2000" der Firma Hans Pausch Röntgengerätebau GmbH + Co., 91056 Erlangen, ist das Röntgengerät "UROMAT 2000" bekannt. Bei dem bekannten Röntgengerät ist eine über einen Motor mit Getriebe vertikal verfahrbare Einrichtung vorgesehen. Die Einrichtung weist einen weiteren Motor zum Verschwerken eines daran gehaltenen Röntgentischs auf. - Die bekannte Vorrichtung ist aufwendig herzustellen. Sie erfordert z.B. das Vorsehen eines Getriebes.

US 4 912 754 A beschreibt eine Vorrichtung zum Bewegen eines Röntgentisches, bei der ein Querhaupt im Eingriff mit zwei Kulissensteinen ist; jeder Kulissenstein greift in das Gewinde einer angetrieben Spindel ein, so dass bei Drehbewegung einer Spindel sich der entsprechende kulissenstein linear bewegt.

In US 5 042 487 wird eine Vorrichtung zum Bewegen der Röntgenquelle eines Computertomographen beschrieben; diese Vorrichtung weist vier angetriebene Spindeln auf, die vier, jeweils in einer Gleitführung aufgenommene Kulissensteine senkrecht bewegen.

Aufgabe der Erfindung ist es, eine möglichst einfach und kostengünstig herstellbare Vorrichtung zum Bewegen eines Röntgentischs oder dgl. anzugeben.

Diese Aufgabe wird durch die Merkmale des Anspruchs 1 gelöst. Zweckmäßige Weiterbildungen der Erfindung ergeben sich aus den Merkmalen der Ansprüche 2 bis 13.

Nach Maßgabe der Erfindung ist eine Vorrichtung zum Bewegen eines Röntgentischs oder dgl. vorgesehen, wobei ein Rotationsmittel im Eingriff mit einem ersten Stellelement einer ersten Linearbewegungseinrichtung und einem parallel dazu bewegbaren zweiten Stellelement einer zweiten Linearbewegungseinrichtung ist, und das Rotationsmittel durch eine unterschiedliche Bewegungsgeschwindigkeit und/oder -richtung des ersten und zweiten Stellelements drehbar ist. - Die Vorrichtung ist einfach aufgebaut. Ein Getriebe ist nicht mehr erforderlich.

Nach einem Ausgestaltungsmerkmal steht die Drehachse des Rotationsmittels im wesentlichen senkrecht zur Bewegungsrichtung der Stellelemente. Das Rotationsmittel kann bei gleicher Bewegungsgeschwindigkeit und -richtung der Stellelemente linear bewegbar sein. Sofern die Stellelemente vertikal bewegbar sind, kann das Rotationsmittel auf einfache Weise vertikal auf und ab bewegt werden.

Zweckmäßigerweise weisen die erste Linearbewegungseinrichtung einen in das Gewinde einer ersten angetriebenen Spindel eingreifenden ersten Kulissenstein und die zweite Linearbewegungseinrichtung einen in das Gewinde einer zweiten angetriebenen Spindel eingreifenden zweiten Kulissenstein auf. Das Rotationsmittel kann eine Achse aufweisen, an deren einem Ende ein Querhaupt vorgesehen ist. Zweckmäßigerweise ist der erste Kulissenstein in einer an einem ersten Ende des Querhaupts vorgesehenen ersten Gleitführung und der zweite Kulissenstein in einer am zweiten Ende des Querhaupts vorgesehene zweiten Gleitführung verschiebbar aufgenommen. Das ermöglicht eine Drehbewegung des Querhaupts bei unterschiedlicher Bewegungsgeschwindigkeit und/oder -richtung der ersten und zweiten Stellelemente. Diese Ausgestaltung hat den Vorteil selbsthemmend zu sein, d.h. bei Stromausfall wird jegliche weitere Bewegung gestoppt.

Am anderen Ende der Achse kann ein Mittel, insbesondere ein Flansch, zur Befestigung eines Röntgentischs, Röntgentischträgers oder dgl. vorgesehen sein. Um bei horizontaler Anordnung der Achse das durch das Gewicht eines daran befestigten Röntgentischs oder Röntgentischträgers bedingte Kippmoment aufzunehmen, ist vorteilhafterweise die Achse in einem linear verfahrbaren Schlitten gelagert. Der Schlitten ist zweckmäßigerweise an zwei parallel stehenden vertikalen Rahmenholmen aufgenommen.

Um eine Bewegung des Röntgentischs in eine vorgegebene Position zu ermöglichen, ist/sind zweckmäßigerweise der erste und/oder der zweiten Kulissenstein mit einem Wegaufnehmer versehen, so daß die Position und/oder der Drehwinkel der Achse ermittelbar ist/sind. Ferner kann mindestens an einem der der Achse abgewandten Enden der Gleitführungen ein durch den ersten bzw. zweiten Kulissenstein betätigbares Schaltelement vorgesehen sein. Dadurch kann die Drehbewegung der Achse automatisch gestoppt werden.

Nach einem weiteren Ausgestaltungsmerkmal bildet bei senkrecht zu den Spindeln stehendem Röntgentisch das Querhaupt einen Winkel vom 25 bis 40°, vorzugsweise 35°, gegenüber einer Röntgentischebene. Dadurch kann die Drehbewegung des Röntgentischs an die praktischen Bedürfnisse angepaßt werden. Der Röntgentisch kann zweckmäßigerweise in der Röntgentischebene bewegbar auf einem Röntgentischträger aufgenommen sein.

Die folgenden Ausführungen betreffen ein Ausgestaltungsmerkmal, welches sowohl in Kombination mit der vorerwähnten Erfindung als auch für sich genommen als weitere Erfindung zu sehen ist: Im Röntgentischträger kann ein Röntgenbildverstärker aufgenommen sein. Der Röntgenbildverstärker ist in der Nähe der einen Querseite des Röntgentischträgers vorgesehen. Das ermöglicht eine besonders einfache Durchleuchtung des Patienten, wenn dieser in der Nähe der einen Querseite des Röntgentischträgers sitzt. Die besonders vorteilhafte Anordnung des Röntgenbildverstärkers wird dadurch ermöglicht, daß eine Filmkasettenaufnahme im Röntgentischträger parallel zu dessen Längsseite verfahrbar ist. Eine Öffnung zum Be- und Entladen der Filmkasettenaufnahme befindet sich in der Nähe der anderen Querseite an der vorderen Längsseite des Röntgentischträgers.

Nachfolgend wird ein Ausführungsbeispiel der Erfindung anhand der Zeichnung näher erläutert. Hierin zeigen
- Fig. 1: eine erste perspektivische Ansicht der Vorrichtung,
- Fig. 2: eine zweite perspektivische Ansicht der Vorrichtung,
- Fig. 3: eine erste Seitenansicht der Vorrichtung mit Rahmen,
- Fig. 4: eine zweite Seitenansicht nach Fig. 3 und
- Fig. 5: eine Draufsicht nach Fig. 3.

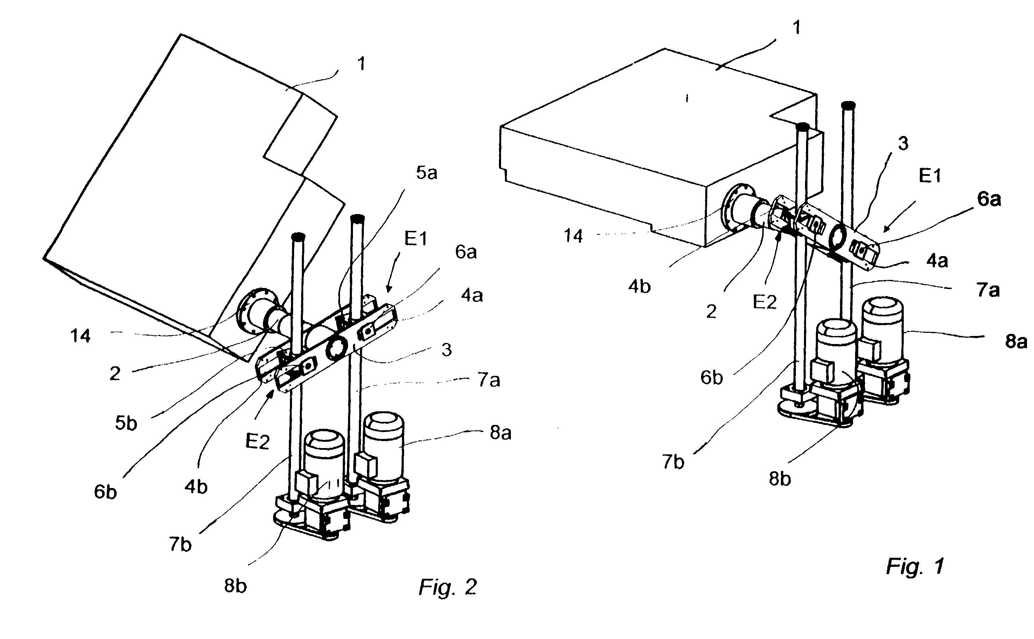

Die Fig. 1 und 2 zeigen pespektivische Ansichten der Vorrichtung ohne Rahmen. Ein Röntgentischträger 1 ist über eine Achse 2 mit einem Querhaupt 3 verbunden. Das Querhaupt 3 besteht hier aus zwei parallelen Platten, an deren ersten E1 und zweiten Enden E2 jeweils erste 4a und zweite Gleitführungen 4b vorgesehen sind. In den Gleitführungen 4a, 4b sind verschiebbar erste 6a und zweite Gleitelemente 6b geführt. Die ersten Gleitelemente 6a sind drehbar an einem ersten Kulissenstein 5a und die zweiten Gleitelemente 6b an einem zweiten Kulissenstein 5b angebracht.

Der erste Kulissenstein 5a ist im Eingriff mit dem Gewinde einer ersten Spindel 7a; der zweite Kulissenstein 5b ist im Eingriff mit dem Gewinde einer zweiten Spindel 7b. Ein erster 8a Elektromotor ist mittels eines Zahnriemens mit der ersten 7a, ein zweiter Elektromotor mittels eines weiteren Zahnriemens mit der zweiten Spindel 7b verbunden.

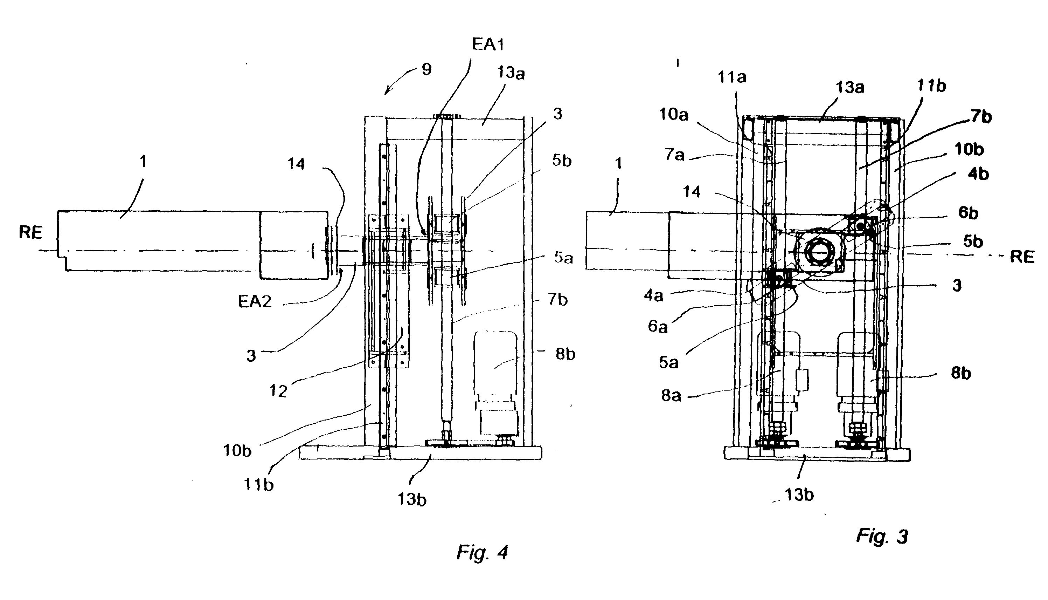

Die Fig. 3 bis 5 zeigen Seitenansichten und eine Draufsicht der Vorrichtung nach Fig. 1 und 2, wobei diese in einem Rahmen 9 aufgenommen ist. An zwei Vorderhol men 10a, 10b des Rahmens 9 sind Führungsschienen 11a, 11b befestigt, in denen ein Schlitten 12 verfahrbar geführt ist. Die Achse 3 ist im Schlitten 12 gelagert. Die erste 7a und die zweite Spindel 7b sind in einem Ober- 13a und einem Unterholm 13b des Rahmens 9 drehbar gelagert. Am einen Ende EA1 ist die Achse 2 mit dem Querhaupt 3 verschweißt. Am anderen Ende EA2 der Achse 2 ist ein Flansch 14 vorgesehen, an dem der Röntgentischträger 1 montiert ist.

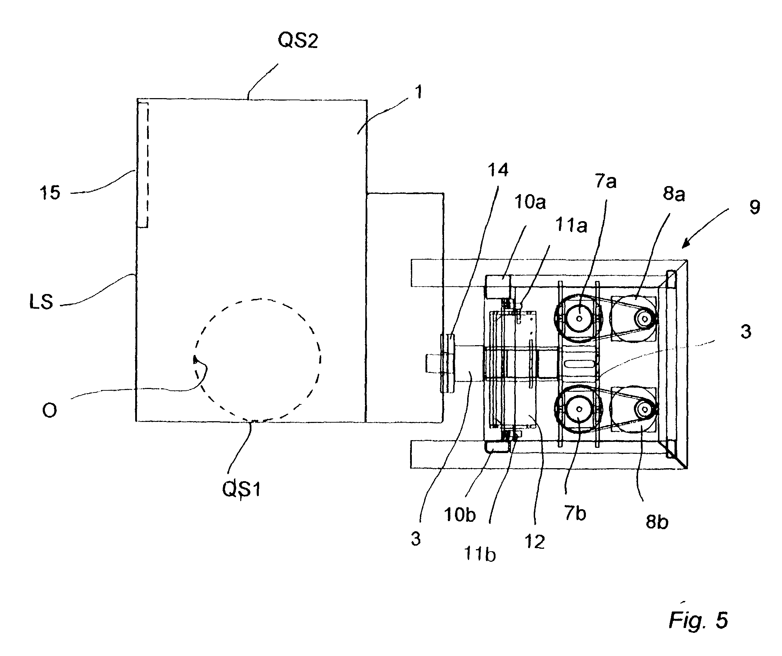

Wie aus Fig. 5 ersichtlich ist, weist der Röntgentischträger 1 eine erste Querseite QS1 und eine zweite Querseite QS2 sowie eine vordere Längsseite LS auf. Im Röntgentischträger 1 ist eine Öffnung O zur Aufnahme eines Röntgenbildverstärkers vorgesehen. In der Nähe der zweiten Querseite QS2 der vorderen Längsseite LS ist eine Be- und Entladeöffnung 15 für Filmkasetten angeordnet. Oberhalb des Röntgentischträgers 1 ist eine (hier nicht gezeigte) Röntgenquelle vorgesehen.

Die Funktion der Vorrichtung ist folgende:

Bei einer gleichsinnigen Drehung der Spindeln 7a und 7b mit derselben Drehzahl werden die drehfest im Querhaupt 3 gehaltenen Kulissensteine 5a, 5b mit derselben Geschwindigkeit vertikal auf- bzw. abwärts bewegt.

Wenn eine der beiden Spindeln 7a oder 7b mit entgegengesetztem Drehsinn und/oder einer anderen Drehzahl als die andere Spindel 7b oder 7a bewegt wird, verschiebt sich der damit in Eingriff stehende Kulissenstein 5a bzw. 5b relativ zum anderen Kulissenstein 5b bzw. 5a. Folglich wird das Querhaupt 3 gedreht. Die dabei bewirkte Änderungen des Abstands zwischen den beiden Kulissensteinen 5a und 5b wird durch die Gleitbewegung der Gleitelemente 6a und 6b in den Gleitführungen 4a und 4b kompensiert.

Im gezeigten Ausführungsbeispiel bildet das Querhaupt 3 bei senkrecht zu den Spindeln 7a, 7b stehender Röntgentischebene RE einen Winkel von etwa 35°. Damit ist es möglich, den Röntgentischträger 1 in Anpassung an die praktischen Bedürfnisse in die eine Richtung um etwa 20° und in die andere Richtung um etwa 80° zu verschwenken. Am Röntgentischträger 1 ist (hier nicht gezeigt) ein Röntgentisch aufgenommen. Der Röntgentisch ist in der Röntgentischebene RE verschiebbar.

### Bezugszeichenliste

- 1: Röntgentischträger
- 2: Achse
- 3: Querhaupt
- 4a, 4b: erste, zweite Gleitführung
- 5a, 5b: erster, zweiter Kulissenstein
- 6a, 6b: erstes, zweites Gleitelement
- 7a, 7b: erste, zweite Spindel
- 8a, 8b: erster, zweiter Elektromotor
- 9: Rahmen
- 10a, 10b: Vorderholme
- 11a, 11b: Führungsschienen
- 12: Schlitten
- 13a: Oberholm
- 13b: Unterholm
- 14: Flansch
- 15: Be- und Entladeöffnung

- E1: erstes Ende
- E2: zweites Ende
- RE: Röntgentischebene
- EA1: erstes Ende der Achse
- EA2: zweites Ende der Achse
- QS1: erste Querseite
- QS2: zweite Querseite
- LS: vordere Längsseite

## Patentansprüche

1. Vorrichtung zum Bewegen eines Röntgentischs oder dgl.,
wobei ein Rotationsmittel eine Achse (2) aufweist, an deren einem Ende (EA1) ein Querhaupt (3) und an deren anderem Ende (EA2) ein Mittel zur Befestigung eines Röntgentischs, Röntgentischträgers (1) oder dgl. vorgesehen ist,
wobei das Querhaupt (3) im Eingriff mit einem in das Gewinde einer ersten angetriebenen Spindel (7a) eingreifenden ersten Kulissenstein (5a) und einem parallel dazu bewegbaren in das Gewinde einer zweiten angetriebenen Spindel (7b) eingreifenden zweiten Kulissenstein (5b) ist,
so dass bei unterschiedlichem Drehsinn und/oder Drehzahl der Spindeln (7a, 7b) eine Drehung des Querhaupts (3) um die Achse (2) erfolgt,
**dadurch gekennzeichnet, dass**
der erste Kulissenstein (5a) in einer an einem ersten Ende (E1) des Querhaupts (3) vorgesehenen ersten Gleitführung (4a) und der zweite Kulissenstein (5b) in einer am zweiten Ende (E2) des Querhaupts (3) vorgesehenen zweiten Gleitführung (4b) verschiebbar aufgenommen ist,

2. Vorrichtung nach Anspruch 1, wobei die Drehachse des Querhaupts (3) im wesentlichen senkrecht zur Bewegungsrichtung der Kulissensteine (5a, 5b) steht.

3. Vorrichtung nach Anspruch 1 oder 2, wobei das Querhaupt (3) bei gleicher Bewegungsgeschwindigkeit und -richtung der Kulissensteine (5a, 5b) linear bewegbar ist.

4. Vorrichtung nach einem der vorhergehenden Ansprüche, wobei das Mittel zur Befestigung ein Flansch (4) ist.

5. Vorrichtung nach einem der vorhergehenden Ansprüche, wobei die Achse (2) in einem linear verschiebbaren Schlitten (12) gelagert ist.

6. Vorrichtung nach einem der vorhergehenden Ansprüche, wobei der erste (5a) und/oder der zweite Kulissenstein (5b) mit einem Wegaufnehmer versehen ist/sind, so daß die Position und/oder der Drehwinkel der Achse (2) ermittelbar ist/sind.

7. Vorrichtung nach einem der vorhergehenden Ansprüche, wobei mindestens an einem der der Achse (2) abgewandten Enden der Gleitführungen (4a, 4b) ein durch die Verschiebebewegung des ersten bzw. zweiten Kulissensteins (5a, 5b) betätigbares Schaltelement vorgesehen ist.

8. Vorrichtung nach einem der vorhergehenden Ansprüche, wobei bei senkrecht zu den Spindeln (7a, 7b) stehendem Röntgentisch (1) das Querhaupt (3) einen Winkel von 25 bis 40°, vorzugsweise von 35°, gegenüber einer Röntgentischebene (RE) bildet.

9. Vorrichtung nach einem der vorhergehenden Ansprüche, wobei ein auf dem Röntgentischträger (1) aufgenommener Röntgentisch in der Röntgentischebene (RE) bewegbar ist.

10. Vorrichtung nach einem der vorhergehenden Ansprüche, wobei im Röntgentischträger (1) eine parallel zu dessen Längsseite verfahrbare Filmkasettenaufnahme vorgesehen ist.

## Claims

1. Device for moving an X-ray table or the like, wherein a rotation means (3) has an axle (2) on the one end (EA1) thereof a crosshead (3) and on the other end (EA2) thereof a means for fastening and X-ray table, X-ray table support (1) or the like is provided,
wherein the crosshead (3) is in engagement with a first sliding block (5a) engaging in a thread of a first driven screw (7a), and with a second sliding block (5b) being movable in parallel to it and engaging in a thread of a second driven screw (7b),
in such manner that by a different rotating direction and/or rotating speed of the driven screws (7a, 7b) a rotation of the crosshead (3) around the axle (2) is caused
**characterised in that**
the first sliding block (5a) is accommodated slideably in a first slideway (4a) provided at a first end (E1) of the crosshead (3) and the second sliding block (5b) is slideably accommodated in a second slideway (4b) at a second end (E2) of the crosshead (3).

2. Device according to claim 1, wherein the axis of rotation of the crosshead (3) is essentially perpendicular to the direction of movement of the sliding blocks (5a, 5b).

3. Device according to claim 1 or 2, wherein the crosshead (3) can be moved linearly at the same speed of movement and direction of movement of the actuators (5a, 5b).

4. Device according to one of the preceding claims, wherein the means for fastening is a flange (4).

5. Device according to one of the preceding claims, wherein the axle (2) is mounted in a linearly displaceable carriage (12).

6. Device according to one of the preceding claims, wherein the first sliding block (5a) and/or the second sliding block (5b) is/are provided with a displacement sensor, with the result that the position and/or the angle of rotation of the axle (2) can be determined.

7. Device according to one of the preceding claims, wherein a control element which can be actuated by the displacement movement of the first or second sliding block (5a, 5b) is provided at least at one of the ends of the slideways (4a, 4b) averted from the axle (2).

8. Device according to one of the preceding claims, wherein with the X-ray table (1) perpendicular to the screws (7a, 7b), the crosshead (3) forms an angle of 25 to 40°, preferably of 35°, with respect to an X-ray table plane (RE).

9. Device according to one of the preceding claims, wherein an X-ray table held on the X-ray table support (1) can be moved in the X-ray table plane (RE).

10. Device according to one of the preceding claims, wherein there is provided in the X-ray table support (1) a film cassette holder which can be moved parallel to the longitudinal side thereof.

## Revendications

1. Dispositif destiné au déplacement d'une table radiologique ou équipement semblable,
un organe de rotation présentant un axe (2) à l'extrémité duquel (EA1) est prévue une traverse (3) et à l'autre extrémité (EA2) un moyen de fixation d'une table radiologique, d'un support radiologique (1) ou équipement semblable,
la traverse (3) étant en prise avec un premier coulisseau (5a) engagé dans le filet d'une première broche (7a) actionnée et avec un deuxième coulisseau (5b), déplaçable en parallèle, engagé dans le filet d'une deuxième broche (7b) actionnée,
**caractérisé par le fait que** le premier coulisseau (5a) peut être déplacé dans une première glissière (4a) prévue sur une première extrémité (E1) de la traverse (3) et que le deuxième coulisseau (5b) peut être déplacé dans une deuxième glissière (4b) prévue sur une deuxième extrémité (E2) de la traverse (3)
de façon qu'une rotation de la traverse (3) soit effectuée autour de l'axe (2) à sens de rotation et/ou vitesse de rotation différent des broches (7a, 7b).

2. Dispositif selon la revendication 1, l'axe de rotation de la traverse (3) étant principalement verticalement par rapport au sens de déplacement des coulisseaux (5a, 5b).

3. Dispositif selon la revendication 1 ou 2, la traverse (3) pouvant être déplacé linéairement, à vitesse et sens de mouvement égaux des coulisseaux (5a, 5b).

4. Dispositif selon une des revendications précédentes, le moyen de fixation étant une bride (4).

5. Dispositif selon une des revendications précédentes, l'axe (2) étant logé sur un chariot (12) déplaçable linéairement.

6. Dispositif selon une des revendications précédentes, le premier (5a) et/ou le deuxième coulisseau (5b) étant muni(s) d'un capteur de course de façon que la position et/ou l'angle de rotation de l'axe (2) puisse(nt) être déterminé(s).

7. Dispositif selon une des revendications précédentes, un élément de commutation actionnable par le déplacement du premier ou du deuxième coulisseau (5a, 5b) étant prévu au moins sur l'une des extrémités des glissières (4a, 4b) opposées à l'axe (2).

8. Dispositif selon une des revendications précédentes, la traverse (3) étant telle qu'elle forme un angle de 25 à 40°, de préférence de 35°, par rapport à un plan de table radiologique (RE), avec une table radiologique (1) verticale aux broches (7a, 7b).

9. Dispositif selon une des revendications précédentes, une table radiologique logée sur un support de table radiologique (1) pouvant être déplacée dans le plan de table radiologique (RE) .

10. Dispositif selon une des revendications précédentes, un logement de cassettes de films déplaçable étant prévu dans le support de table radiologique (1) parallèlement à son grand côté.
